# EUROPÄISCHE PATENTSCHRIFT

(11) **EP 1 941 891 B1**
(45) Veröffentlichungstag und Bekanntmachung des Hinweises auf die Patenterteilung: **13.02.2013**
(21) Anmeldenummer: 06799670.2
(22) Anmeldetag: 04.09.2006
(51) Int. Cl.: A61K 35/74, A61K 31/635, A61K 31/606, A61P 1/04

(54) **ZUBEREITUNG ZUR BEHANDLUNG VON NICHT-INFEKTIÖSEN ENTZÜNDLICHEN DARMERKRANKUNGEN**
PREPARATION FOR TREATING NON-INFECTIOUS INFLAMMATORY INTESTINAL DISEASES
PREPARATION DESTINEE A TRAITER DES MALADIES INTESTINALES INFLAMMATOIRES NON INFECTIEUSES

(30) Priorität: 26.09.2005 RU 2005129581
(43) Veröffentlichungstag der Anmeldung: 09.07.2008
(73) Patentinhaber: Zakrytoe Akcionernoe Obshhestvo "Partner", Moscow 117303 (RU)
(72) Erfinder: VAYNSHTOK, Igor Izmailovich, Moscow, 115470 (RU); MACULEVICH, Tamara Vasilevna, Moscow, 121353 (RU); BOLOTOV, Valeriy Dmitrievich, Moscow, 113405 (RU); DOROSHENKO, Ekaterina Olegovna, Moscow, 105037 (RU)
(74) Vertreter: Jeck, Anton
(86) Internationale Anmeldenummer: PCT/RU2006/000471
(87) Internationale Veröffentlichungsnummer: WO 2007/035129

(56) Entgegenhaltungen:
- EP-A1- 1 547 601
- WO-A-01/11334
- WO-A-2005/074908
- RU-A- 2001 119 046
- RU-C2- 2 181 043
- RU-C2- 2 228 184
- US-A- 5 519 014
- BOUSVAROS ATHOS ET AL: "A randomized, double-blind trial of Lactobacillus GG versus placebo in addition to standard maintenance therapy for children with Crohn's disease." INFLAMMATORY BOWEL DISEASES SEP 2005, Bd. 11, Nr. 9, September 2005 (2005-09), Seiten 833-839, XP009120895 ISSN: 1078-0998
- TURSI A ET AL: "Mesalazine and/or Lactobacillus casei in preventing recurrence of symptomatic uncomplicated diverticular disease of the colon: A prospective, randomized, open-label study" JOURNAL OF CLINICAL GASTROENTEROLOGY, RAVEN PRESS LTD., NEW YORK, NY, US, Bd. 40, Nr. 2, 1. April 2006 (2006-04-01), Seiten 312-316, XP009112141 ISSN: 0192-0790
- VENTURI A ET AL: "Impact on the composition of the faecal flora by a new probiotic preparation: Preliminary data on maintenance treatment of patients with ulcerative colitis" ALIMENTARY PHARMACOLOGY AND THERAPEUTICS, Bd. 13, Nr. 8, August 1999 (1999-08), Seiten 1103-1108, XP002539551 ISSN: 0269-2813
- DATABASE PUBMED [Online] 02 March 2005 KLOTZ U.: 'Colonic targeting of aminosalicylates for the treatment of ulcerative colitis', XP004888107 Database accession no. (15893274) & DIG. LIVER DIS. vol. 37, no. 6, June 2005, pages 381 - 388

## Beschreibung

Die Erfindung betrifft die Medizin und kann zur Behandlung von nicht infektiösen, entzündlichen Darmkrankheiten verwendet werden.

Die nicht infektiösen, entzündlichen Darmkrankheiten, zu denen die geschwürartige Kolitis und die Crohn's-Krankheit gehören, sind eines der schwersten Probleme moderner Gastroenterologie. Die Häufigkeit einer geschwürartigen Kolitis und der Crohn's-Krankheit wächst in der ganzen Welt mit jedem Jahr, vorzugsweise unter dem arbeitsfähigen Teil der Bevölkerung. Dies macht diese Erkrankungen sozial bedeutend.

Zur Behandlung der nicht infektiösen, entzündlichen Darmkrankheiten werden die Präparate der 5-Aminosalicylsäure, nämlich Sulfasalazin und Mesalazin, verwendet.

Bekannt ist das Präparat Sulfasalzin (Enzyklopädie der Medikamente, "Das Register der medikamentösen Mittel Russlands/RLS", Moskau, OOO RLS-2005, 2004, 12. Ausgabe, S. 826-827). Es hat eine bakterien- und entzündungshemmende Wirkung. Im Bindegewebe des Darms zerfällt es in 5-Aminosalicylsäure (5-ASA) und Sulphapyridin. Die 5-ASA bedingt die entzündungshemmenden Eigenschaften von Sulphasalazin. Sulphapyridin ist ein konkurrierender Antagonist der Paraamino-Benzoesäure, stellt die Folatsynthese in den Zellen der Mikroorganismen ein und bedingt die antibakterielle Aktivität. Der Nachteil des Präparats bei der Behandlung der geschwürartigen Kolitis und der Crohn's-Krankheit ist eine starke Abhängigkeit vom Zustand der Darmnormalflora, die an der Biotransformation von Sulfasalazin und der Freisetzung der aktiven Komponente 5-ASA teilnimmt. Die zweite Komponente des Präparats Sulphapyridin unterdrückt die Bakterien, bremst tatsächlich und hält die spezifische Aktivität des Präparats aufrecht. Im Zusammenhang damit verstärken sich wahrscheinlich allergische Auswirkungen und diskinetische Störungen drastisch, und die Leberfunktion wird gestört.

Bekannt ist das Präparat Salofalk. Dies sind Tabletten mit einem Überzug, der sich im Darm auflöst. Die Tabletten enthalten Mesalazin-5-ASA >Enzyklopädie der Medikamente, "Das Register der medikamentösen Mittel Russlands RLS", Moskau, OOO RLS-2005, 2004, 12. Ausgabe, S. 780-781). Das Präparat ist ein entzündungshemmendes Mittel, verhindert die Synthese der Arachidonsäuremetaboliten und die Aktivität der neutrophilen Lipooxigenase, hemmt die Migration, Degranulation und Phagozitose der neutrophilen Granulozyten sowie die Sekretion von Immunglobilinen durch Lymphozyten und bindet und zerstört freie Radikale des Sauerstoffs. Es wird zur Behandlung der geschwürartigen Kolitis und der Crohn's-Erkrankung verschrieben. Die Nachteile des Präparats sind ausgeprägte, allergische Auswirkungen, Funktionsstörungen des Darms, Nerven-, Herz-Kreislauf- und Urinsystems, die als Nebenwirkungen auftreten. Die Anwendung des Präparats wird nur unter Arztkontrolle und mit regelmäßigen Blut- und Urinuntersuchungen empfohlen.

Bekannt ist auch eine pharmazeutische Zusammensetzung zur oralen Verabreichung. Sie enthält 5-ASA als aktiven Bestandteil. Sie ist zur modifizierten Freisetzung aus dem Überzug zur Aufrechterhaltung eines klinisch wichtigen, lokalisierten und wirksamen Profils von 5-ASA im Dünn- und Dickdarm angepasst (RU 2181043). Zu den Nachteilen kann auch das Folgende gezählt werden. Zur Auswirkung der pharmakologischen Aktivität der 5-ASA wird ein genügend aktiver Zustand der Normalflora benötigt, die neben der allgemeinen, entgiftenden Wirkung an der Acetylierung der 5-ASA und deren Ausscheidung aus dem Darm teilnimmt. Der allgemeine Zustand der Normalflora und insbesondere deren anaeroben Glieder (Bifido- und Lactobakterien) ist aber noch vor der spezifischen Behandlung stark gehemmt und einer obligatorischen Korrektur besonders bei Rückfällen bedürftig.

Bousvaros A. et. al., (Inflammatory Bowl Disease, Bd. 11, Nr. 9, September 2005, Seiten 833 bis 839) betrifft die Untersuchung einer Kombinationstherapie von Morbus Crohn mit 5-Aminosalizylat und probiotischen Bakterien.

Die Aufgabe der Erfindung besteht in der Schaffung eines Präparats zur Behandlung entzündlicher Darmerkrankungen, das nicht die genannten Nachteile aufweist und Bakterien enthält, die für die Normalflora des Magen-Darm-Trakts charakteristisch sind.

Dies wird durch die Gegenstände der unabhängigen Ansprüche erreicht.

Das technische Ergebnis der Erfindung besteht in einer Wirksamkeitserhöhung der Behandlung von nicht infektiösen, entzündlichen Darmkrankheiten und in einer Kürzung der Behandlungszeit.

Die genannte Aufgabe wird dadurch gelöst, dass das Präparat zur Behandlung der entzündlichen Darmerkrankungen, das 5-Aminosalicylsäure oder einen Stoff, der im Organismus mit der Bildung der 5-Aminosalicylsäure zerfällt, eine lyophil- getrocknete Mikrobenmasse von lebenden Bifido- oder Lactobakterien bzw. deren Mischung mit der folgenden Zusammensetzung der Komponenten zusätzlich enthält:

| 5- Aminosalicylsäure oder den Stoff, der im Organismus | |
|---|---|
| mit der Bildung der 5-Aminosalicylsäure zerfällt: | 0,05 - 1,00 g, |
| lyophil-getrocknete Mikrobenmasse | |
| von lebenden Bakterien | 10⁵- 10¹⁰ KBE. |

Das Präparat enthält Bifidobakterienoder Lactobakterien oder deren Mischung, die auf feindisperser aktivierter Kohle oder Siliziumdioxid immobilisiert sind.

Das Präparat kann Bifidobakterien oder Bifidobakterien enthalten, die auf einem Sorbens immobilisiert sind.

Das Präparat kann Lactobakterien oder Lactobakterien enthalten, die auf einem Sorbens immobilisiert sind.

Das Präparat kann eine Mischung von Bifido- und Lactobakterien oder eine Mischung von auf einem Sorbens immobilisierten Bifido- und Lactobakterien enthalten.

Als 5-Aminosalicylsäure oder Stoff, der im Organismus mit der Bildung von 5-Aminosalicylsäure zerfällt, kann das Präparat Mesalazin oder Sulfasalazin enthalten. Mesalazin oder Sulphasalazin kann als Pulver oder Granulat enthalten sein.

Die Komponenten des Präparats können in zwei Kapseln gruppiert sein, die zusammen eine Einmaldosis bilden, wobei eine Kapsel eine Mikrobenmasse lebender Bakterien und die andere Mesalazin oder Sulfasalazin enthält.

Die Komponenten können auch in zwei Kapseln gruppiert sein, wobei eine Kapsel eine Mikrobenmasse lebender Bakterien enthält, während in der anderen Kapsel eine Mesalazin und Sulphasalazin enthaltende Kapsel untergebracht ist.

Das Wesen der Erfindung besteht im Folgenden.

Es bestehen zahlreiche Theorien und Annahmen über die Entstehungsgründe der nicht infektiösen, entzündlichen Darmkrankheiten, und zwar eine Regulationsstörung des Immunsystems im Darminneren, Autoimmunprozesse, eine genetische Empfänglichkeit für die Einwirkung der Mikrobentoxine oder für eine nicht adäquate Antwort des Immunsystems usw.

Daten der letzten Jahre und diesseitige Forschungen zeugen von dem Vorhandensein tiefer mikroökologischer Störungen im Verdauungstrakt bei den Kranken mit einer geschwürartigen Kolitis und Crohn's-Erkrankung. Dies ermöglicht zu vermuten, dass diesen Darmerkrankungen ein Gleichgewichtsfehler im ökologischen System "der Wirt und seine Mikroflora" zugrunde liegt. Dies wird auch durch die Feststellung einer Zustandsbesserung der Patienten bei der Verschreibung speziell ausgewählter Probiotika bestätigt, die die Zusammensetzung der Darmflora normalisieren.

Die mikroökologische Analyse des Gehalts im unteren Bereich des Dünndarms bei den chronischen entzündlichen Verletzungen des Verdauungstrakts deckt die Vergrößerung des Verhältnisses von potentiell pathogenen, aeroben Enterobakterien, Bakteroiden und anderen anaeroben, gramnegativen Bakterien sowie grampositiven, aeroben und anaeroben Kokken auf. Ein ähnliches Bild ist auch bei der Untersuchung des Gehalts des Dickdarms vorhanden.

Ein grundsätzlich wichtiger Parameter einer herabgesetzten Kolonisationsresistenz des Verdauungstrakts bei chronischen Darmentzündungen ist eine intensive Verkleinerung der Bifidoflora und Lactobazillen im Darmgehalt, die natürliche Antagonisten der potentiell pathogenen Mikroorganismen sind, und eine Vergrößerung des Verhältnisses von Bakteroiden, anaeroben, grampositiven Kokkenbakterien und aeroben Enterobakterien. Das Verhältnis der Zahl der Anaerobier und Aerobier in der parietalen Flora sinkt intensiv und erreicht das Verhältnis 25:1.

Der quantitative Gehalt von Bakteroiden, Bifidobakterien, Eubakterien, Peptokokken, Streptokokken, Enterobakterien und organischen Säuren im Kot der Menschen, die an geschwürartiger Kolitis leiden, unterscheidet sich bei kranken und gesunden Menschen wesentlich.

Eine vergleichende Untersuchung des Gehalts von Lactobazillen, Bifidobakterien und Bakteroiden in Exkrementen von gesunden Menschen und von Personen mit der Crohn's-Krankheit in der Rückfallphase zeigte eine heftige Verkleinerung der Menge an Bifidobakterien bei den Kranken. Die Senkung der Bifidobakterienanzahl geschieht parallel mit einem wesentlichen Rückgang der Aktivität der summarischen, fäkalen β-Galaktosidase.

Die durchgeführten Untersuchungen bestätigten, dass die Präparate, die 5-ASA oder den Stoff enthalten, der im Organismus mit der Bildung der 5-ASA zerfällt, ihre entzündungshemmende Aktivität nach der Transformation der Mikroben im Dickdarm entwickeln. Dabei wird die freigesetzte 5-ASA nur nach bakterieller Spaltung aktiv.

Außerdem spielt in der Pathogenese nicht spezifischer, geschwürartiger Kolitis und der Crohn's-Krankheit die Mikroflorastörung im Dickdarm eine wichtige Rolle. Die Rolle der Immunstörungen bei diesen Erkrankungen ist nachgewiesen.

Die eingesetzten Bifido- und Lactobakterien sind natürliche und obligatorische Bewohner des Darms. Sie sind ungiftig, nicht virulent, nicht giftig für Menschen und Tiere, verfügen über eine hohe, antagonistische Aktivität zu pathogenen und bedingt pathogenen Mikroorganismen und verhindern deren Adhäsion an der Schleimhaut des Darms. Sie tragen zur Normalisierung der Tätigkeit des Magen-Darm-Trakts und Erhöhung einer nicht spezifischen Resistenz des Organismus bei, aktivieren parietale Verdauung, synthesieren Aminosäuren, Vitamine und weisen eine immunmodulierende Wirkung auf.

Nach Angaben klinischer Tests weisen hohe Dosen von Bifidobakterien (insbesondere die Präparate Probifor, Bifidumbakterin) sowie Lactobakterien oder deren Mischungen eine ausgeprägte Wirkung auf Reparaturprozesse in der Schleimhaut des Dickdarms auf und tragen zum ausgeprägten Untergehen der Entzündung darin bei. Die Anwendung dieser Bakterien trägt zur schnellen Wiederherstellung der Mikrobiozönose des Darms, der Verbesserung der Prozesse der parietalen Verdauung und des Aufsaugens bei und verbessert die Funktionen des Immunsystems.

Die gemeinsame Anwendung der Normalflorabakterien und der 5-ASA ermöglicht, allgemeine toxische und allegorische Auswirkung wesentlich zu verringern, und trägt zur Stabilisierung und der Wiederherstellung der natürlichen Darmmikroflora bei, wodurch wiederum eine Erhöhung der Wirksamkeit von 5-ASA durch eine synchrone Transformation der Mikroben (Acetylieren) erreicht wird.

Der Gehalt an 5-ASA oder dem Stoff, der im Organismus mit der Bildung von 5-ASA zerfällt, entspricht innerhalb von 0,05 - 1,00 g der therapeutischen Dosis, die gewöhnlich den Patienten zur Behandlung der geschwürartigen Kolitis oder der Crohn's-Krankheit vorgeschrieben wird.

Ein Gehalt an lypophil-getrockneter Mikrobenmasse lebender Bakterien in einer Größe von weniger als 10⁵ KBE erzeugt keine Heilwirkung, da er bei gestörter Mikroökologie des Darms die benötigte Kolonisationsaktivität und antagonistische Wirksamkeit nicht gewährleistet

Ein Gehalt an lypophil-getrockneter Mikrobenmasse lebender Bakterien in einer Größe über 10¹⁰ ist unzweckmäßig, da er zu einer ungerechtfertigten Mehrausgabe der Biomasse führt und die Behandlung verteuert.

Die Verwendung der auf einem Sorbens immobolisierten Bakterien gewährleistet eine hohe, lokale Kolonisierung der Darmschleimhaut und verstärkt die Wiederaufbauprozesse darin. Als Sorbenzien werden feindisperse, aktivierte Kohle oder Siliziumdioxid verwendet.

Als 5-ASA oder Stoff, der im Organismus mit der Bildung von 5-ASA zerfällt, können die vorzugsweise Mesalazin und Sulfasalazin enthaltenden Präparate verwendet werden. Es können auch andere Aminosalizylate, die 5-ASA nach der chemischen Struktur nahe stehen und nach dem Wirkungsmechanismus analog sind, insbesondere Olsalazin, Balsalazin usw., verwendet werden.

Mesalazin oder Sulfasalazin als Pulver ist zur Aufbereitung von Mikroklysmen und zur rektalen Verabreichung sowie peroral als Granulat zweckmäßigerweise zu verwenden.

Für eine bequeme Verabreichung sowie zur Sicherung der Auflösungsmöglichkeit der Komponenten des Präparats in bestimmten Bereichen des Magen-Darm-Trakts können die Komponenten in zwei Kapseln gruppiert werden, die zusammen eine Einmaldosis bilden, wobei eine Kapsel die Mikrobenmasse lebender Bakterien und die andere Mesalazin oder Sulfasalazin enthält.

Die Komponenten können auch in zwei Kapseln gruppiert werden, wobei eine Kapsel die Mikrobenmasse lebender Bakterien enthält und in der anderen eine Mesalazin oder Sulfasalazin aufweisende Kapsel untergebracht wird.

Die Erfindung wird nun anhand der folgenden Beispiele näher erläutert, wobei die Beispiele 1 bis 6 die Zusammensetzung des Präparats gemäß der Erfindung charakterisieren.

### Beispiel 1

| | |
|---|---|
| 5-ASA, Pulver, Lyophilisierte Masse lebender | 0,05 g, |
| Bifidobakterien | 5x10⁸ KBE. |

### Beispiel 2

| | |
|---|---|
| 5-ASA, Pulver Lyophilisierte Masse lebender | 0,10 g, |
| Bifido- und Lactobakterien | 10⁸ KBE. |

### Beispiel 3

| | |
|---|---|
| 5-ASA, Pulver Lyophilisierte Masse lebender, | 0,25 g, |
| sorbierter Lactobakterien KBE, | 10⁶ KBE. |

### Beispiel 4

| | |
|---|---|
| 5-ASA, Granula Lyophilisierte Masse lebender, | 0,50 g, |
| sorbierter Bifidobakterien | 5x10⁷ KBE. |

### Beispiel 5

| | |
|---|---|
| 5-ASA, Granula Lyophilisierte Masse lebender | 0,75 g, |
| Lactobakterien | 10⁸ KBE. |

### Beispiel 6

| | |
|---|---|
| 5-ASA, Granula Lyophilisierte Masse lebender, | 1,00 g, |
| sorbierter Bifido- und Lactobakterien | 5 x 10⁹ KBE. |

### Beispiel 7 (Einschätzung der Unschädlichkeit des Präparats in Tierversuchen)

Bei den vorklinischen Versuchen wurde die Unschädlichkeit der angebotenen Rezeptur und deren einzelner Komponenten in Versuchen mit weißen Mäusen eingeschätzt. Zu diesem Zweck wurde eine für die medizinische, immunbiologische Präparate übliche Methode gewählt. Die Untersuchungen wurden mit Mäusen mit einem Gewicht von höchstens 15 g durchgeführt, die sich von den erwachsenen Mäusen durch eine erhöhte Sensibilität zur Wirkung der alimentären Faktoren unterscheiden. Im Laufe von fünf Tagen der Beobachtung wurden Veränderungen des Gewichts und alle Merkmale der Erkrankung registriert. Die Verkleinerung des Gewichts, das Erscheinen der Merkmale der Erkrankung oder der Tod der Tiere zeugen von der schädlichen Einwirkung auf den Organismus der Mäuse.

Nach dem Parameter "Unschädlichkeit" wurden probiotische Komponenten des Präparats, Bifido- und Lactobakterien in Höhe von nicht weniger als 10⁸ KBE und ein Mischung der probiotischen Komponenten mit Sulfasalazin in Dosen von 50, 100 und 150 mg pro 1 kg des Gewichts des Tiers eingeschätzt. Die Ergebnisse der Untersuchungen zeigten auch, dass die getrennt genommenen, probiotischen Komponenten bei der Verabreichung direkt in den Magen der weißen Mäuse unschädlich ist; der Zusatz von Sulfasalazin zu dem Probiotikum in der Dosis von 50 und 100 mg/kg hatte keine Wirkung auf die Unschädlichkeit; die Vergrößerung der Sulfasalazin-Dosis bis zu 150 mg/kg ermöglichte, die Merkmale der Schädlichkeit (Verkleinerung des Gewichts der Mäuse um 10 %) aufzuzeigen.

Somit wird die Dosis des Präparats, die 100 mg 5-ASA und nicht weniger als 10⁸ Probiotikum pro 1 kg des Gewichts der Tiere enthält und die therapeutische Tagesdosis für einen Menschen übertrifft, von den Tieren gut vertragen.

Die Beispiele 8 bis 10 charakterisieren die Wirksamkeit des Präparats bei der Behandlung der geschwürartigen Kolitis und der Crohn's-Krankheit.

### Beispiel 8

Dem Patienten C., 28 Jahre, wurde die klinische Diagnose: nicht spezifische geschwürartige Kolitis, Totalverletzung hoher Aktivität, Rückfallform gestellt. Die Anamnese ergab eine Dauer der Erkrankung von 5 Jahren. Die Krankenhausunterbringung betrug während des Rückfalls 40 - 60 Tage. Bei der ärztlichen Untersuchung ist ein Rückfall in eine nicht spezifische, geschwürartige, mittelschwere Kolitis festgestellt worden. Klinisch: Stuhlhäufigkeit 4 - 6 mal pro Tag, mäßige Blutbeimischung, subfebrile Temperatur, keine Pathologie seitens des Herz-Kreislaufsystems, Herzfrequenz bis 90 Herzschlägen pro Minute, BSG 30 mm/h, mäßige Leukozytose, unbedeutende Malabsorption, Darmschmerzen im Ruhezustand und bei Palpieren. Die Endoskopie ergab eine diffuse Hyperämie, eine Körnigkeit und ein Ödem der Schleimhaut, kein Gefäßbild, mäßiges Bluten, plurale Erosion, einzelne Geschwüre, Fibrin und keinen Eiter.

Eine mikrobiologische Kotuntersuchung ergab eine Störung der Mikrobiozönose-Senkung des Gehalts an Bifidobakterien bis 10⁷ KBE/g, eine Senkung des Gehalts an Lactobakterien bis 10⁵ KBE/g, eine Senkung der Menge der Darmstäbchen bis 7 x 10⁶ KBE/g und eine Vergrößerung der Kokkenformen in der Gesamtmenge der Mikroorganismen bis 95 % (Norm bis 25 %).

Bei der komplexen Behandlung bekam der Patient das Präparat, das 4 g/Tag 5-ASA und 5 x 10⁸ Bifidobakterien auf einem Sorbens nach dem Beispiel 4 enthielt, im Laufe von 21 Tagen. Vor der Entlassung wurde nach der durchgeführten Therapie eine positive Dynamik-Remission nicht spezifischer, geschwürartiger Kolitis festgestellt. Die Klinikwerte ergaben eine normale Stuhlhäufigkeit, einen Stuhl ohne pathologische Beimischungen, eine normale Körpertemperatur, keine Tachikardie und keine Darmschmerzen. Die positive Dynamik des Endoskopiebilds zeigte eine unbedeutende Erythema der Schleimhaut, Lockerheit und ein rarefiziertes Gefäßbild. CRP (C-reaktives Protein) war negativ, Hämoglobin und BSG waren innerhalb der Norm.

Die ausgeprägte, positive Dynamik nach den Daten der mikrobiologischen Kotuntersuchung zeigte einen normalen Gehalt an Bifidobakterien (10⁸/g), einen normalen Gehalt an Lactobakterien (10⁶/g), eine Erhöhung des Gehalts an Darmstäbchen im Verhältnis zur Untersuchung vor der Behandlung (10⁷/g) und eine Senkung der Menge der Kokkenformen in der Gesamtmenge der Mikroorganismen (50 %).

Somit ist der Rückfall in die geschwürartige Kolitis in 21 Tagen verschwunden. Der Patient wurde in einem befriedigenden Zustand entlassen.

Eine Verabreichung des Präparats, das 5-ASA in einer Tagesdosis bis 1 g gleichzeitig mit sorbiertem Probiotikum enthielt, wurde empfohlen.

### Beispiel 9

Dem Patienten L., 58 Jahre, wurde eine nicht spezifische, geschwürartige Kolitis, distale Form (chronische geschwürartige Proktitis) und mittelschwer klinisch diagnosiziert. Anamnese: der Patient ist im Laufe von 28 Jahren mit Rückfällen und vorübergehenden Besserungen krank. Während eines Rückfalls wurde er immer im Krankenhaus untergebracht. Der mittlere Aufenthalt im Krankenhaus betrug bis 40 Tage. Eine Hämokolitis wurde gewöhnlich in der 2. - 3. Woche beseitigt.

Eine ärztliche Untersuchung zeigte einen Rückfall in eine nicht spezifische, geschwürartige, mittelschwere Kolitis. Beim klinischen Aufenthalt zeigte bzw. zeigten sich eine Stuhlhäufigkeit bis 6 mal pro Tag mit Schleim und Blut, ein blutigtingierter Kot, ein subfebriles Fieber, Bauch- und Defäkationsschmerzen, mäßig ausgeprägte Änderungen auf dem Elektrokardiogramm, eine Tachikardie bis 85 Herzschlägen pro Minute, ein Gewichtsverlust von etwa 4 kg in den letzten 6 Monaten, ein BKS von 43 mm/h, mäßige Leukozytose und eine mäßig ausgeprägte Malabsorption.

Eine Endoskopie ergab eine diffuse Hyperämie, eine Körnigkeit und ein Ödem der Schleimhaut, kein Gefäßbild, ein mäßiges Bluten, eine plurale Erosion, einzelne Geschwüre, Fibrin und eine geringe Eitermenge.

Eine mikrobiologische Kotuntersuchung ergab eine Störung der Mikrobiozönose-Senkung des Gehalts an Bifidobakterien bis 10⁷ KBE/g und eine Senkung des Gehalts an Lactobakterien bis 10⁵ KBE/g.

Bei der komplexen Behandlung bekam der Patient das Präparat, das 4 g/Tag 5-ASA und 10⁸ Lactobakterien auf einem Sorbens nach dem Beispiel 5 enthielt. Eine Besonderheit des Behandlungsschemas bei diesem Patienten war, dass das Präparat 5-ASA gleichzeitig mit Lactobakterien verabreicht wurde. Die Behandlung dauerte 25 Tage. Die Hämokolitis verschwand am 6. Tag der Behandlung.

Nach der durchgeführten Behandlung waren keine Schmerzen im Darm vorhanden; die Stuhlhäufigkeit war innerhalb der Norm, und der Stuhl war ohne pathologische Beimischungen; der Patient hatte normale Temperatur und keine Tachikardie.

Eine Endoskopie ergab ein unbedeutendes Erythema der Schleimhaut und ein rarefiziertes Gefäßbild. Die BKS war bis auf 18 mm/Stunde gesunken. Nach den Daten der mikrobiologischen Kotuntersuchung wurde keine Mikrobiozenöse des Darms beobachtet; der Gehalt an Bifidobakterien betrug 10⁷/g und der Gehalt an Lactobakterien betrug 10⁷/g.

Der Patient wurde in einem befriedigenden Zustand entlassen. Eine ambulatorische Behandlung im Laufe von 4 Monaten mit einer Verabreichung des 5-ASA enthaltenden Präparats in einer Dosis von 1 g pro Tag und mit sorbierten Bifidobakterien wurde empfohlen.

### Beispiel 10

Dem Patienten R., 30 Jahre, wurde die Crohn's Krankheit diagnostiziert. Mit einer Körpertemperatur von 37,5 °C, mit Beschwerden in Form von heftiger Schwäche, mit Störungen des Muskeltonus, Muskel-, Bauch- und Gelenkschmerzen, Diarrhoe und Gewichtverlust wurde er ins Krankenhaus aufgenommen.

Die Anamnese ergab keine Erkrankungen des Verdauungstraktes. Es bestand aber ein Verdacht auf überstandene Yersiniose. In diesem Zusammenhang wurde der Patient auf Antikörper gegen unterschiedliche bakterielle und virale Infektionserreger einschließlich Yersinia, Campylobakter und Candida untersucht. Erkrankungen des Verdauungstraktes waren ausgeschlossen.

Bei der Kolon- und Gastroskopie wurden Gastritis, Duodenitis, herdförmige, katarrhalische Kolitis und Disbakteriose festgestellt. Eine Untersuchung im Krankenhaus ergab eine blasse Haut und Schleimhaut, Tachikardie, eine erhöhte Körpertemperatur, einen angeschwollenen Bauch, mäßige Bauchschmerzen im linken Bereich des Krummdarms und einen spasmierten Sigmadarm bei tiefer Bauchpalpation. Eine Diarrhoe trat 4 - 5 mal am Tag auf, ferner ein unförmiger Stuhl mit Blut-, Schleim- und Eiterbeimischung, eine Leukozytose und eine erhöhte BSG. Bei der biochemischen Untersuchung wurden herabgesetzte Konzentration von Kalium, Natrium und Magnesium im Blutserum, eine erhöhte Kreatinphosphokinase, eine erhöhte Sättigung mit Eisen, Alpha- und Gammaglobulin sowie Beta-2-Mikroglobulin registriert. Ein niedriger Albumingehalt im Blut lag vor.

Bei der bakteriologischen Kotuntersuchung auf Disbakteriose wurde eine Senkung der Menge an Darmstäbchen mit der normalen, funktionalen Aktivität, an Enterokokken und an Bifido- und Lactobakterien festgestellt.

Bei der komplexen Behandlung wurde das Präparat nach Beispiel 6 verschrieben, das 5-ASA in einer Höhe von 4 g/Tag und 10⁸ KBE in der Mischung sorbierter Bifido- und Lactobakterien enthielt, jeweils dreimal am Tag je einen Beutel. Die Behandlung dauerte 21 Tage.

Nach der durchgeführten Behandlung verbesserte sich der Zustand des Patienten wesentlich. Die Stuhlhäufigkeit war einmal am Tag ohne pathologische Beimischungen, und die Bauchschmerzen waren verschwunden. Bei der bakteriologischen Kotuntersuchung auf Disbakteriose wurde eine ausgeprägte, positive Dynamik festgestellt. Die Menge an Bifido- und Lactobakterien, normalen Darmstäbchen und Enterokokken hatte zugenommen.

Somit ist das Präparat gemäß der Erfindung bei der Behandlung der nicht infektiösen, entzündlichen Darmerkrankungen wirksam und ermöglicht, die Behandlungsdauer zu verringern.

## Patentansprüche

1. Präparat zur Behandlung nicht infektiöser, entzündlicher Darmkrankheiten, das die 5-Aminosalicylsäure oder einen Stoff enthält, der im Organismus mit der Bildung der 5-Aminosalicylsäure zerfällt,
**dadurch gekennzeichnet,**
**dass** es lyophyl-getrocknete Mikrobenmasse lebender Bifido- oder Lactobakterien oder deren Mischung, die auf feindisperser aktivierter Kohle oder Siliziumdioxid immobilisiert sind, mit folgendem Verhältnis der Komponenten, pro therapeutischer Dosis, zusätzlich enthält:
- 5- Aminosalicylsäure oder den Stoff, der im Organismus mit der Bildung der 5-Aminosalicylsäure zerfällt 0,05 - 1,00 g
- und lyophyl-getrocknete Mikrobenmasse von lebenden Bakterien 10⁵ -10¹⁰ KBE.

2. Präparat zur Behandlung nicht infektiöser, entzündlicher Darmkrankheiten nach Anspruch 1,
**dadurch gekennzeichnet,**
**dass** es 5-Aminosalicylsäure oder einen Stoff, der im Organismus mit der Bildung von 5- Aminosalicylsäure zerfällt, und zwar Mesalazin oder Sulfasalazin, enthält.

3. Präparat zur Behandlung nicht infektiöser, entzündlicher Darmerkrankungen nach Anspruch 1,
**dadurch gekennzeichnet,**
**dass** es Mesalazin oder Sulfasalazin als Pulver oder Granulat enthält.

4. Präparat zur Behandlung nicht infektiöser, entzündlicher Darmerkrankungennach einem der Ansprüche 1 bis 3,
**dadurch gekennzeichnet,**
**dass** die Komponenten in zwei Kapseln gruppiert sind, die zusammen eine einmalige Dosis bilden, wobei eine Kapsel die Mikrobenmasse lebender Bakterien und die andere Mesalazin oder Sulfasalazin enthält.

5. Präparat zur Behandlung nicht infektiöser, entzündlicher Darmerkrankungen nach einem der Ansprüche 1 bis 3,
**dadurch gekennzeichnet,**
**dass** die Komponenten in zwei Kapseln gruppiert sind, wobei eine Kapsel die Mikrobenmasse lebender Bakterien und die andere eine Mesalazin oder Sulfasalazin aufweisende Kapsel enthält.

## Claims

1. A preparation for treating noninfectious, inflammatory intestinal diseases, which contains 5-aminosalicylic acid or a substance that decomposes in the organism, forming 5-aminosalicylic acid,
**characterized in that**
it additionally contains lyophil-dried microbial biomass of live bifido- or lactobacteria or mixtures thereof, which are immobilized on finely dispersed activated charcoal or silicon dioxide, with the following proportion of the components per therapeutic dose:
- 5-aminosalicylic acid or the substance that decomposes in the organism, forming 5-aminosalicylic acid, 0.05 - 1.00 g
- and lyophil-dried microbial biomass of live bacteria, 10⁵- 10¹⁰ cfu.

2. The preparation for treating noninfectious, inflammatory intestinal diseases according to claim 1,
**characterized in that**
it contains 5-aminosalicylic acid or the substance that decomposes in the organism, forming 5-aminosalicylic acid, specifically mesalazine or sulfasalazine.

3. The preparation for treating noninfectious, inflammatory intestinal diseases according to claim 1,
**characterized in that**
it contains mesalazine or sulfasalazine as a powder or granulate.

4. The preparation for treating noninfectious, inflammatory intestinal diseases according to one of claims 1-3,
**characterized in that**
the components are grouped in two capsules which together form a single dose, and one capsule contains the microbial biomass of live bacteria and the other contains mesalazine or sulfasalazine.

5. The preparation for treating noninfectious, inflammatory intestinal diseases according to one of claims 1-3,
**characterized in that**
the components are grouped in two capsules, and one capsule contains the microbial biomass of live bacteria and the other contains a capsule having mesalazine or sulfasalazine.

## Revendications

1. Préparation destinée au traitement de maladies intestinales inflammatoires non infectieuses, qui contient l'acide 5-aminosalicylique ou une substance qui se décompose dans l'organisme avec formation d'acide 5-aminosalicylique, **caractérisée en ce qu'**elle contient une substance microbienne lyophilisée de bifidobactéries ou de lactobactéries vivantes ou de leur mélange, qui sont immobilisées sur du charbon actif ou du dioxyde de silicium finement dispersé, avec le rapport suivant des composants, par dose thérapeutique :
- de l'acide 5-aminosalicylique ou une substance qui se décompose dans l'organisme avec formation d'acide 5-aminosalicylique 0,05 -1,00 g
- et une substance microbienne lyophilisée de bactéries vivantes 10⁵ - 10¹⁰ UFC.

2. Préparation destinée au traitement de maladies intestinales inflammatoires non infectieuses selon la revendication 1, **caractérisée en ce qu'**elle contient de l'acide 5-aminosalicylique ou une substance qui se décompose dans l'organisme avec formation d'acide 5-aminosalicylique, à savoir de la mésalazine ou de la sulfasalazine.

3. Préparation destinée au traitement de maladies intestinales inflammatoires non infectieuses selon la revendication 1, **caractérisée en ce qu'**elle contient de la mésalazine ou de la sulfasalazine sous la forme d'une poudre ou de granulés.

4. Préparation destinée au traitement de maladies intestinales inflammatoires non infectieuses selon l'une des revendications 1 à 3, **caractérisée en ce que** les composants sont groupés dans deux capsules qui forment conjointement une dose unitaire, une capsule contenant la substance microbienne de bactéries vivantes et l'autre contenant la mésalazine ou la sulfasalazine.

5. Préparation destinée au traitement de maladies intestinales inflammatoires non infectieuses selon l'une des revendications 1 à 3, **caractérisée en ce que** les composants sont groupés dans deux capsules, une capsule contenant la substance microbienne de bactéries vivantes et l'autre contenant une capsule présentant la mésalazine ou la sulfasalazine.
